# EUROPEAN PATENT SPECIFICATION

(11) **EP 3 612 112 B1**
(45) Date of publication and mention of the grant of the patent: **01.06.2022**
(21) Application number: 18788166.9
(22) Date of filing: 19.04.2018
(51) Int. Cl.: A61B 17/80

(54) **SELF-ADJUSTABLE PECTUS RECONSTRUCTION SYSTEM**
SELBSTEINSTELLBARES PECTUSREKONSTRUKTIONSSYSTEM
SYSTÈME DE RECONSTRUCTION DE PECTUS AUTO-RÉGLABLE

(30) Priority: 20.04.2017 TW 106113333; 05.07.2017 US 201715641358
(43) Date of publication of application: 26.02.2020
(73) Proprietor: Su, Sea-Quan, 112 Taipei (TW); Su, Susan Bing, Irvine, California 92618 (US)
(72) Inventor: SU, Sea-Quan, 112 Taipei (TW)
(74) Representative: Papula Oy
(86) International application number: PCT/US2018/028239
(87) International publication number: WO 2018/195235

(56) References cited:
- WO-A1-2017/023147
- CN-U- 203 693 730
- CN-U- 204 600 644
- CN-U- 205 379 321
- US-A1- 2010 256 691
- US-A1- 2011 251 540
- US-A1- 2012 130 371

## Description

### 1. Technical Field

The present disclosure provides a self-adjustable pectus reconstruction system having a self-adjustable structure that can be self-stretching when a patient breathes and providing an expanded space to avoid growth limitation.

### 2. Description of Related Art

Pectus excavatum is a congenital disease because of an abnormal hyperplasia of cartilago costalis on the both sides of sternum and leads to a concave sternum; when the symptom is severe, it will oppress the heart and lungs leading to abnormal conditions such as dyspnea, chest pain, cardiopulmonary dysfunction and the like. Commonly, cardiopulmonary dysfunction is less found in childhood, whereas it becomes severe in adolescence.

A traditional treatment is to remove the deformation of cartilago costalis so as to regenerate cartilago costalis and elevate the concave sternum simultaneously. Nowadays, the traditional treatment is replaced by a minimally invasive surgery; a tailor-made metal plate is placed behind the sternum in order to push out the concave sternum and cartilage costalis without resection surgery, and the metal plate should be retained in the body for at least 2 to 4 years before removal.

CN 203693730U discloses an integrated memory alloy funnel chest orthosis which belongs to the technical field of a medical instrument and is used for treating thoracic wall funnel chest malformation. The integrated memory alloy funnel chest orthosis mainly comprises a memory alloy orthopedic plate 1, fixed plates 2 and hinge pins 6; both ends of the memory alloy orthopedic plate 1 are provided with waist-shaped holes 5; the middles of the fixed plates 2 are provided with pin holes 4; the middle positions at one ends of the fixed plates 2 are provided with straight slots 9 in the thickness direction; two fixed plates 2 respectively pass through both ends of the memory alloy orthopedic plate 1 by the straight slots 9 and then pass through the pin holes 4 and the waist-shaped holes 5 by the hinge pins 6 so as to be connected with both ends of the memory alloy orthopedic plate 1. The orthosis has good material biocompatibility and a light weight. The product is simple for the surgical operation, is safe to use, is comfortable and safe to wear and has a good orthopedic effect.

CN 205379321U provides an orthopedic steel sheet in scalable funnel thorax, including a first steel sheet and a second steel sheet and having a sheath mouth and a connecting utricle of the sheath tail; the one end of the first steel sheet connects with the sheath tail-to-tail linking, and the other end of the first steel sheet extends a butterfly wing. The first round hole and second round hole have been set up to butterfly wing both ends symmetry, and the sheath mouth is equipped with the stopper. The slot has been set up to the one end of the second steel sheet, and the stopper is arranged in to follow in the slot slides. The third round hole has been set up to the other end of the second steel sheet. The utility model provides an improvement time in 3 years that can be guaranteed along with teenagers' body development automatically regulated to orthopedic steel sheet in scalable funnel thorax length, reaching the correction purpose and not influencing teenagers' eutaxy again and growth.

US 2011/251540A1 discloses an apparatus and method for treating pectus excavatum. A pectus bar stabilizer having two or more channels may be used to secure two or more pectus bars within the chest cavity of a patient. The channels of the pectus bar stabilizer may be spaced apart to support pectus bars that are positioned one intercostal space apart or more than one intercostal space apart.

CN 204600644U relates to a kind of novel funnel chest and corrected steel plate, and the described funnel chest is corrected steel plate and comprised of T-shaped plate, curved plate, and fixed strip; the described T-shaped plate comprises telescopic block and fixed block; two sides of the described telescopic block are provided with a flange, and the described telescopic block is connected with a fixed block by an arc section; one end of the described curved plate is provided with a chimeric block, and the described chimeric block and curved plate junction are provided with indicatrix; one end of the described curved plate medial surface is provided with groove; the described fixed block and telescopic block form a chimeric cavity; the described chimeric cavity matches with the chimeric block on the curved plate; two ends of the described fixed block and chimeric cavity central authorities are equipped with a through hole, and the through hole on its chimeric block corresponds with the through hole on chimeric cavity. Its advantage shows: on the basis of NUSS operation, simplifying operation technique further, shortening operating time, raising surgical effect and success rate, alleviating patient's misery, saving surgery cost, and shortening the hospital stays.

However, most of the orthopedic internal fixation implants cannot automatically adjust the length, size, and position to conform to human growth or the respiratory changes without affecting clinical functions; therefore, it causes discomfort after implantation and even needs re-implantation to conform to patient's body growth. Accordingly, it is necessary to develop a bone orthopedic appliance which is self-adjustable to conform to patient's growth or respiratory changes.

### SUMMARY

The present invention is defined in claim 1 while preferred embodiments are set forth in the dependent claims.

The present disclosure provides a self-adjustable pectus reconstruction system having a self-adjustable structure that can be self-stretching and reduce the patient's pain and foreign body sensation when the patient breathes. In addition, since many patients receiving pectus excavatum correction surgeries are children and young people, the present disclosure having the self-adjustable structure can provide an expanded space to avoid growth limitation. Meanwhile, the fixing unit of the present disclosure can be arc-shaped for conforming to ergonomics to avoid foreign body sensation and the risk of patient's skin extrusion.

The present disclosure provides a self-adjustable pectus reconstruction system, comprising: a pectus reconstruction bar having a first end and a first slot disposed on the first end; a first fixing unit having a first channel; and a first connector, wherein the first end of the pectus reconstruction bar is assembled on the first channel of the first fixing unit, and the first connector passes through the first slot to combine the pectus reconstruction bar with the first fixing unit, such that the first slot is capable of sliding back and forth around the first connector. The first fixing unit further comprises a first through-hole, and the system is fixed to a tissue by the first through-hole of the first fixing unit. The system further comprises a second fixing unit and a second connector, and the second fixing unit is combined with the pectus reconstruction bar by the second connector. An extended direction of the first slot is parallel to a longitudinal direction of the pectus reconstruction bar. The pectus reconstruction bar comprises an arc-shaped structure to fit patient's shape of sternum.

The present disclosure further provides a self-adjustable pectus reconstruction system, comprising a pectus reconstruction bar having a second end and a second slot disposed on the second end, wherein the second fixing unit has a second channel, and wherein the second end of the pectus reconstruction bar is assembled on the second channel of the second fixing unit, and the second connector passes through the second slot to combine the pectus reconstruction bar with the second fixing unit, such that the second slot is capable of sliding back and forth around the second connector. The second fixing unit further comprises a second through-hole, and the system is fixed to a tissue by the second through-hole of the second fixing unit. An extended direction of the second slot is parallel to a longitudinal direction of the pectus reconstruction bar.

Since the pectus reconstruction system needs to be placed in patient's body for a long period of time, the present disclosure has taken patients' needs into consideration. The self-adjustable pectus reconstruction system provided by the present disclosure has a self-adjustable structure that can adjust its structure when the patient is breathing, and the slot can slide back and forth around the connector according to the movement of sternum, and adjust the length of the system automatically so as to reduce pain and foreign body sensation. In addition, the self-adjustable structure also provides the patient with an expanded space to avoid growth limitation.

In a further embodiment of the present disclosure, the first and second fixing units of the self-adjustable pectus reconstruction system may optionally have an arc-shaped structure for fitting patient's sternum to avoid foreign body sensation. In the self-adjustable pectus reconstruction system, the first fixing unit and the second fixing unit are hermetic to prevent human tissue from migrating into the first fixing unit and the second fixing unit that causes a system failure or difficulty of removing the system afterwards.

In addition, the present disclosure further provides a method for reconstructing pectus excavatum by using the aforementioned self-adjustable pectus reconstruction system. This method does not form part of the present invention.

The method of the present disclosure may comprise the following steps: providing the aforementioned self-adjustable pectus reconstruction system; passing the pectus reconstruction bar behind patient's deformed sternum; rotating the pectus reconstruction bar whereby the deformed sternum is raised into a desired position; and fixing the pectus reconstruction bar onto the patient's bone through the fixing unit.

Other advantages and features of the disclosure will become more apparent from the following detailed description when taken in conjunction with the accompanying drawings.

### BRIEF DESCRIPTION OF THE DRAWINGS

FIG. 1 is a system diagram according to Embodiment 1 of the present disclosure.
FIG. 2 is an assembly diagram according to Embodiment 1 of the present disclosure.
FIG. 3 is a cross sectional view of one end of the system according to Embodiment 1 of the present disclosure.
FIG. 4 is a cross sectional view of one end of the system according to Embodiment 2 of the present disclosure.
FIG. 5 is a schematic diagram of a fixing unit according to Embodiment 3 of the present disclosure.
FIG. 6 is a schematic diagram of a pectus reconstruction bar according to Embodiment 4 of the present disclosure.
FIG. 7 is a schematic diagram showing correction of pectus excavatum according to Embodiment 1 of the present disclosure.
FIG. 8 is a schematic diagram showing correction of pectus excavatum according to Embodiment 1 of the present disclosure.

### DETAILED DESCRIPTION OF THE EMBODIMENT

### Embodiment 1

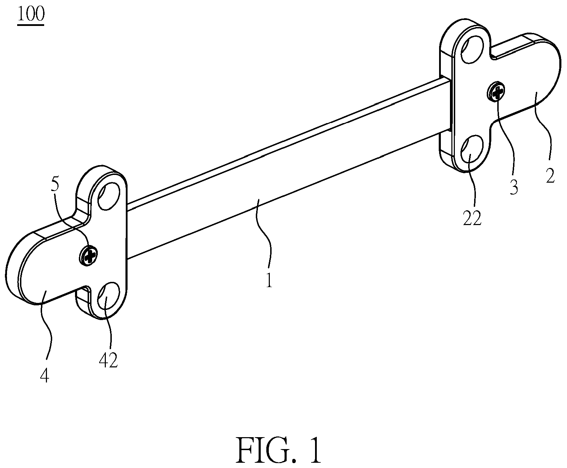

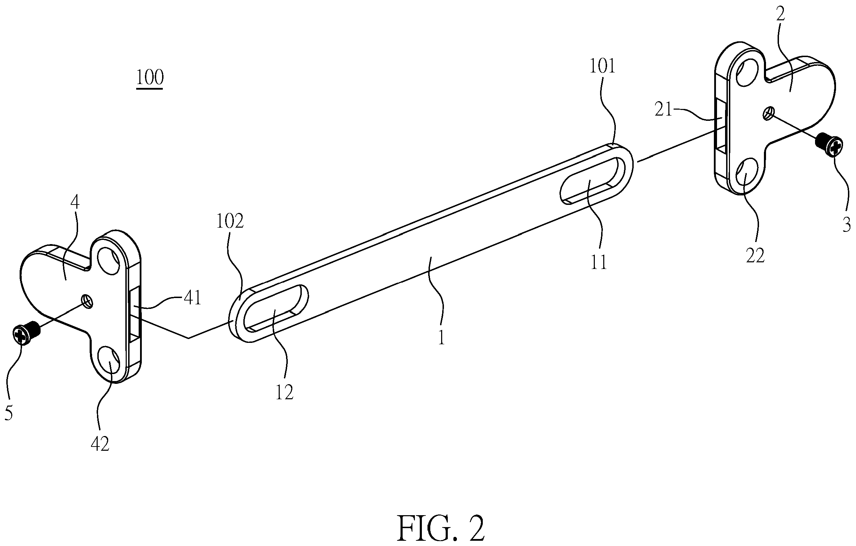

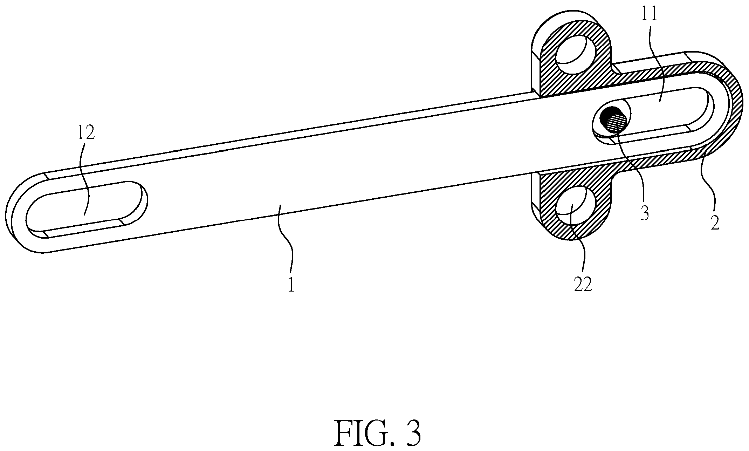

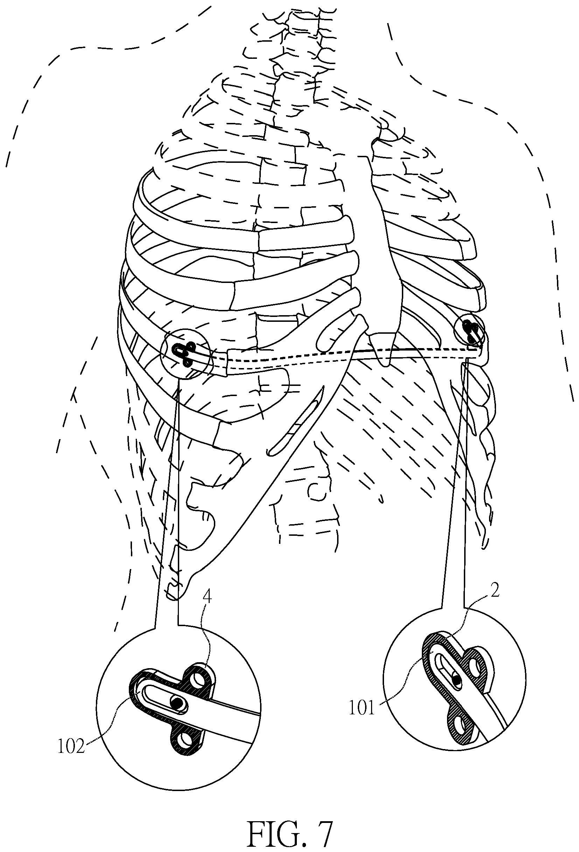

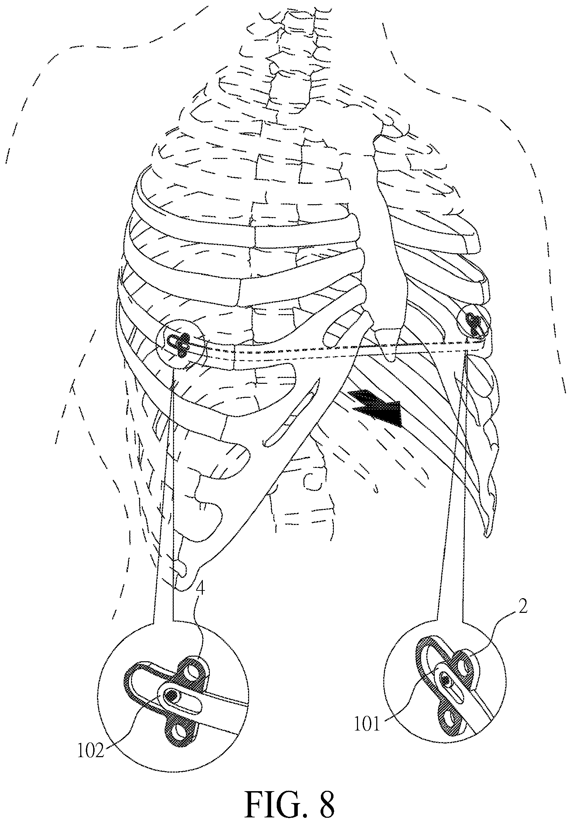

FIG. 1 is a system diagram of the present disclosure; FIG. 2 is an assembly diagram of FIG. 1; FIG. 3 is a cross sectional view of a first end of the system; FIG. 7 is a schematic diagram showing correction of pectus excavatum; and FIG. 8 is another schematic diagram showing correction of pectus excavatum.

A self-adjustable pectus reconstruction system 100 of the present embodiment comprises: a pectus reconstruction bar 1 having a first end 101 and a first slot 11 disposed on the first end 101; a first fixing unit 2 having a first channel 21; and a first connector 3, wherein the first end 101 of the pectus reconstruction bar 1 is assembled on the first channel 21 of the first fixing unit 2, and the first connector 3 passes through the first slot 11 to combine the pectus reconstruction bar 1 with the first fixing unit 2, such that the first slot 11 of the pectus reconstruction bar 1 is capable of sliding back and forth around the first connector 3. The first fixing unit 2 further comprises a first through-hole 22, and the system 100 is fixed to a tissue by the first through-hole 22 of the fixing unit 2. An extended direction of the first slot 11 is parallel to a longitudinal direction of the pectus reconstruction bar 1.

The self-adjustable pectus reconstruction system 100 of the present embodiment further comprises a second fixing unit 4 and a second connector 5. The pectus reconstruction bar 1 further comprises a second end 102 and a second slot 12 disposed on the second end 102, and the second fixing unit 4 has a second channel 41, wherein the second end 102 of the pectus reconstruction bar 1 is assembled on the second channel 41 of the second fixing unit 4, and the second connector 5 passes through the second slot 12 to combine the pectus reconstruction bar 1 with the second fixing unit 4, such that the second slot 12 of the pectus reconstruction bar 1 is capable of sliding back and forth around the second connector 5. Furthermore, an extended direction of the second slot 12 is parallel to a longitudinal direction of the pectus reconstruction bar 1. The second fixing unit 4 further comprises a second through-hole 42, and the system 100 is fixed to a tissue by the second through-hole 42 of the second fixing unit 4.

Referring to FIG.7, it shows a cross sectional view of the first end 101 and the second end 102 of the present embodiment fixed to a human tissue, and the sternum is in its original position when the human body is in an expiratory state. Referring to FIG. 8, it shows a cross sectional view of the first end 101 and the second end 102 of the present embodiment fixed to a human tissue, and the sternum is raised when the human body is in an inspiratory state. Since the present disclosure has a self-adjustable structure, it can adjust its structure when the patient is breathing. The slots of the pectus reconstruction bar 1 can slide back and forth around the connectors according to the movement of sternum, thereby adjusting the length of the system automatically so as to reduce pain and foreign body sensation. In addition, the self-adjustable structure of the present disclosure can also provide the patient with an expanded space to avoid growth limitation. As shown in FIG. 7 and FIG. 8, the first fixing unit 2 and the second fixing unit 4 are illustrated in cross sectional views to clearly show the relative positions between the pectus reconstruction bar 1 and the first fixing unit 2 and the second fixing unit 4. In one embodiment, the first fixing unit 2 and the second fixing unit 4 used in the patient may be represented as shown in FIG. 7 and FIG. 8. In an actual situation, the first fixing unit 2 and the second fixing unit 4 may be represented as shown in FIG. 1 and FIG. 2.

The material of the pectus reconstruction bar 1 may be selected from materials known in the art such as stainless steel, titanium alloy and the like, and the slot may be bar-shaped, e.g., in an elliptical or rectangular shape. The material of the fixing unit may be the same as or different from the material of the pectus reconstruction bar, and may be selected from materials known in the art such as stainless steel, titanium alloy and the like. The connector can be any fixing element. The first fixing unit and the second fixing unit are hermetic to prevent human tissue from migrating into the first and second fixing units that causes a system failure or difficulty of removing the system afterwards.

### Embodiment 2

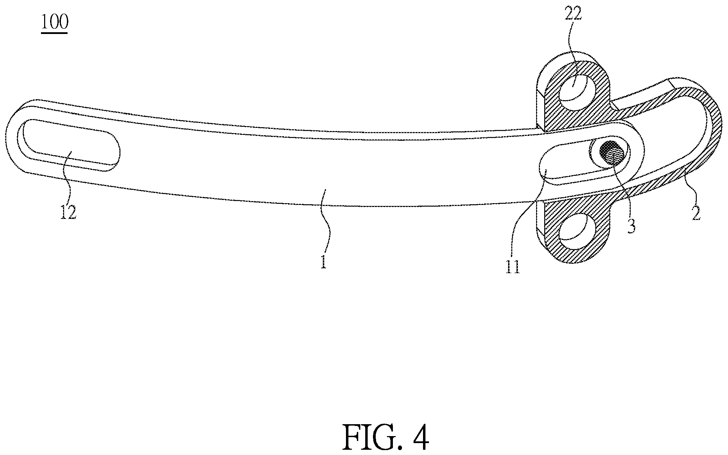

FIG. 4 is a cross sectional view of one end of the system 100 according to the present disclosure, wherein the self-adjustable pectus reconstruction system 100 of the present embodiment is similar to that of Embodiment 1 except for the following differences.

Referring to FIG. 4, in the present embodiment, the first fixing unit 2 and/or the second fixing unit (not shown) may have an arc-shaped structure if necessary. In addition, the pectus reconstruction bar 1 comprises an arc-shaped structure to fit patient's shape of sternum. When the first fixing unit 2, the second fixing unit and/or the pectus reconstruction bar 1 have an arc-shaped structure, the system 100 can fit patient's shape of sternum more to avoid foreign body sensation and the risk of patient's skin extrusion.

### Embodiment 3

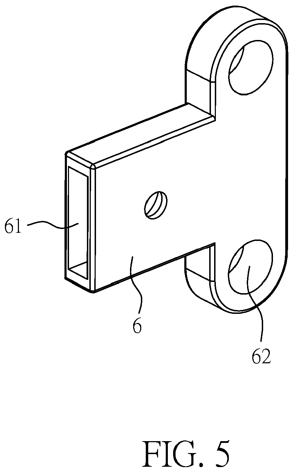

FIG. 5 is a schematic diagram of a fixing unit of the present disclosure. As shown in FIGs. 1 through 4, the first through-hole 22 of the first fixing unit 2 and/or the second through-hole 42 of the second fixing unit 4 can be plural according to the pectus reconstruction system 100 in Embodiment 1 or Embodiment 2. In the present embodiment, the first through-hole 62 of the first fixing unit 6 and/or the second through-hole of the second fixing unit (not shown) can be plural and disposed on both ends of the fixing unit 6. The amount and installation position of the through-holes of the first fixing unit 6 and/or the second fixing unit are not particularly limited, as long as they can fix the system 100 to a tissue.

### Embodiment 4

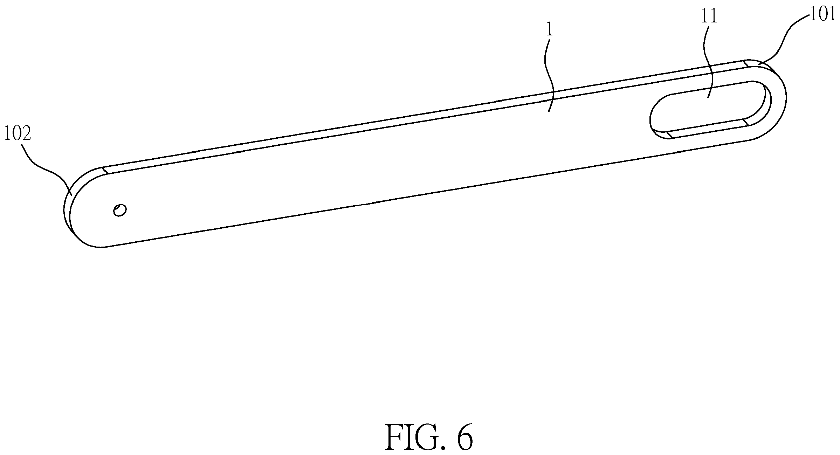

FIG. 6 is a schematic diagram of the pectus reconstruction bar 1 according to the present disclosure, wherein the self-adjustable pectus reconstruction system 100 of the present embodiment is similar to Embodiment 1 except for the following differences.

Referring to FIG. 6, in the present embodiment, the pectus reconstruction bar 1 comprises a first end 101 and a first slot 11 disposed on the first end 101, whereas there is no second slot disposed on the second end 102 of the pectus reconstruction bar 1. Therefore, there is only one self-adjustable structure disposed on one end of the self-adjustable pectus reconstruction system 100 in the present embodiment. Accordingly, it is an aspect of the reconstruction system 100 that one end of the system is self-adjustable, whereas the other end is not self-adjustable.

### Embodiment 5

In this embodiment, a tailor-made pectus reconstruction bar of the reconstruction system is provided according to patient's anterior chest wall curvature. Further, an incision of 1.5 cm to 2 cm long on each side of the lateral chest wall is made between the anterior axillary line and the posterior axillary line, and then the self-adjustable pectus reconstruction bar is inserted behind patient's deformed sternum in order to push out the concave sternum and the deformed cartilage costalis. The pectus reconstruction bar is rotated whereby the deformed sternum is raised into a desired position, and then the bar is fixed onto patient's bone through the fixing unit. The pectus reconstruction system will be retained within the patient for 2-4 years depending on the patient's age and condition, and then be removed after the shape of the sternum is fixed.

In contrast to traditional surgery, the self-adjustable pectus reconstruction system of the present disclosure provides a minimally invasive technique with shorter operating time, smaller incision, less dissection, less sterna fracturing, less pain, quick postoperative recovery, and shorter hospital stay, and retains patient's bone elasticity of the anterior chest wall.

The self-adjustable pectus reconstruction system of the present disclosure can be self-stretching to reduce patient's pain and foreign body sensation when the patient is breathing. It also provides an expanded space to avoid patient's body growth limitation. Further, the fixing unit can be arc-shaped for conforming to ergonomics to avoid foreign body sensation and the risk of skin extrusion.

It should be appreciated that the above embodiments shall be construed as merely illustrative.

## Claims

1. A self-adjustable pectus reconstruction system (100), comprising:
a pectus reconstruction bar (1) having a first end (101) and a first slot (11) disposed on the first end;
a first fixing unit (2) having a first channel (21); and
a first connector (3),
wherein the first end of the pectus reconstruction bar is disposed in the first channel of the first fixing unit, and the first connector passes through the first slot to combine the pectus reconstruction bar with the first fixing unit, wherein
the first slot of the pectus reconstruction bar slides back and forth around the first connector, **characterized in that** the first fixing unit (2) is hermetic to prevent human tissue from migrating into the first fixing unit (2).

2. The system as claimed in claim 1, wherein the first fixing unit further has a first through-hole (22) configured for the system to be fixed to a tissue.

3. The system as claimed in claim 1, wherein an extended direction of the first slot is parallel to a longitudinal direction of the pectus reconstruction bar.

4. The system as claimed in claim 1, wherein the first fixing unit has an arc-shaped structure.

5. The system as claimed in claim 1, further comprising a second fixing unit (4) and a second connector (5),
wherein the second fixing unit is combined with the pectus reconstruction bar (1) by the second connector.

6. The system as claimed in claim 5, wherein the pectus reconstruction bar (1) further has a second end (102) and a second slot (12) disposed on the second end, wherein the second fixing unit (4) has a second channel (41),
and wherein the second end of the pectus reconstruction bar is disposed in the second channel of the second fixing unit, and the second connector passes through the second slot to combine the pectus reconstruction bar with the second fixing unit, such that the second slot of the pectus reconstruction bar slides back and forth around the second connector.

7. The system as claimed in claim 5, wherein the second fixing unit (4) further has a second through-hole (42) configured for the system to be fixed to a tissue.

8. The system as claimed in claim 6, wherein an extended direction of the second slot is parallel to a longitudinal direction of the pectus reconstruction bar.

9. The system as claimed in claim 1, wherein the pectus reconstruction bar has an arc-shaped structure.

10. The system as claimed in claim 5, wherein the second fixing unit has an arc-shaped structure and the second fixing unit is hermetic to prevent human tissue from migrating into the second fixing unit.

11. The system as claimed in claim 1, wherein the first slot is elongated in a lengthwise direction of the pectus reconstruction bar.

12. The system as claimed in claim 11, wherein the first fixing unit extends in the lengthwise direction.

13. The system as claimed in claim 6, wherein the second slot is elongated in a lengthwise direction of the pectus reconstruction bar.

14. The system as claimed in claim 13, wherein the second fixing unit extends in the lengthwise direction.

## Patentansprüche

1. Selbsteinstellbares Pektus-Rekonstruktionssystem (100), umfassend:
einen Pektusrekonstruktionsstab (1) mit einem ersten Ende (101) und einem ersten Schlitz (11), der an dem ersten Ende angeordnet ist;
eine erste Befestigungseinheit (2) mit einem ersten Kanal (21); und
ein erstes Verbindungsstück (3),
wobei das erste Ende des Pektus-Rekonstruktionsstabs in dem ersten Kanal der ersten Fixiereinheit angeordnet ist, und der erste Verbinder durch den ersten Schlitz hindurchgeht, um den Pektus-Rekonstruktionsstab mit der ersten Fixiereinheit zu verbinden, wobei der erste Schlitz des PektusRekonstruktionsstabs um den ersten Verbinder herum hin und her gleitet, **dadurch gekennzeichnet, dass** die erste Fixiereinheit (2) hermetisch ist, um zu verhindern,
dass menschliches Gewebe in die erste Fixiereinheit (2) wandert.

2. System nach Anspruch 1, wobei die erste Fixiereinheit ferner ein erstes Durchgangsloch (22) aufweist, das so konfiguriert ist, dass das System an einem Gewebe befestigt werden kann.

3. System nach Anspruch 1, bei dem die Erstreckungsrichtung des ersten Schlitzes parallel zur Längsrichtung des Pektusrekonstruktionsstabs verläuft.

4. System nach Anspruch 1, wobei die erste Befestigungseinheit eine bogenförmige Struktur aufweist.

5. System nach Anspruch 1, ferner umfassend eine zweite Fixiereinheit (4) und ein zweites Verbindungsstück (5), wobei die zweite Fixiereinheit durch das zweite Verbindungsstück mit dem Pektusrekonstruktionsstab (1) verbunden ist.

6. System nach Anspruch 5, wobei der Pektusrekonstruktionsstab (1) ferner ein zweites Ende (102) und einen zweiten Schlitz (12) aufweist, der an dem zweiten Ende angeordnet ist, wobei die zweite Fixiereinheit (4) einen zweiten Kanal (41) aufweist, und wobei das zweite Ende des Pektusrekonstruktionsstabs in dem zweiten Kanal der zweiten Fixiereinheit angeordnet ist und der zweite Verbinder durch den zweiten Schlitz hindurchgeht, um den Pektusrekonstruktionsstab mit der zweiten Fixiereinheit zu verbinden, so dass der zweite Schlitz des Pektusrekonstruktionsstabs um den zweiten Verbinder herum hin und her gleitet.

7. System nach Anspruch 5, wobei die zweite Fixiereinheit (4) ferner ein zweites Durchgangsloch (42) aufweist, das so konfiguriert ist, dass das System an einem Gewebe befestigt werden kann.

8. System nach Anspruch 6, wobei eine Erstreckungsrichtung des zweiten Schlitzes parallel zu einer Längsrichtung des Pectus-Rekonstruktionsstabs ist.

9. System nach Anspruch 1, wobei der Pektusrekonstruktionsstab eine bogenförmige Struktur aufweist.

10. System nach Anspruch 5, wobei die zweite Befestigungseinheit eine bogenförmige Struktur aufweist und die zweite Befestigungseinheit hermetisch ist, um zu verhindern, dass menschliches Gewebe in die zweite Befestigungseinheit wandert.

11. System nach Anspruch 1, wobei der erste Schlitz in Längsrichtung des Pectus-Rekonstruktionsstabs verlängert ist.

12. System nach Anspruch 11, wobei sich die erste Fixiereinheit in der Längsrichtung erstreckt.

13. System nach Anspruch 6, wobei sich der zweite Schlitz in Längsrichtung des Pektusrekonstruktionsstabs erstreckt.

14. System nach Anspruch 13, wobei sich die zweite Fixiereinheit in Längsrichtung erstreckt.

## Revendications

1. Système de reconstruction de thorax auto-réglable (100), comprenant :
une barre de reconstruction de thorax (1) présentant une première extrémité (101) et une première fente (11) disposée sur la première extrémité ;
une première unité de fixation (2) présentant un premier canal (21) ; et
un premier raccord (3),
dans lequel la première extrémité de la barre de reconstruction de thorax est disposée dans le premier canal de la première unité de fixation, et le premier raccord traverse la première fente pour combiner la barre de reconstruction de thorax avec la première unité de fixation, dans lequel la première fente de la barre de reconstruction de thorax coulisse d'avant en arrière autour du premier raccord, **caractérisé en ce que** la première unité de fixation (2) est hermétique pour empêcher le tissu humain de migrer dans la première unité de fixation (2).

2. Système selon la revendication 1, dans lequel la première unité de fixation présente en outre un premier trou traversant (22) configuré pour que le système soit fixé à un tissu.

3. Système selon la revendication 1, dans lequel une direction étendue de la première fente est parallèle à une direction longitudinale de la barre de reconstruction de thorax.

4. Système selon la revendication 1, dans lequel la première unité de fixation présente une structure en forme d'arc.

5. Système selon la revendication 1, comprenant en outre une seconde unité de fixation (4) et un second raccord (5), dans lequel la seconde unité de fixation (4) est combinée avec la barre de reconstruction de thorax (1) par le second raccord.

6. Système selon la revendication 5, dans lequel la barre de reconstruction de thorax (1) présente en outre une seconde extrémité (102) et une seconde fente (12) disposée sur la seconde extrémité, dans lequel la seconde unité de fixation (4) présente un second canal (41), et dans lequel la seconde extrémité de la barre de reconstruction de thorax est disposée dans le second canal de la seconde unité de fixation, et le second raccord traverse la seconde fente pour combiner la barre de reconstruction de thorax avec la seconde unité de fixation, de telle sorte que la seconde fente de la barre de reconstruction de thorax coulisse d'avant en arrière autour du second raccord.

7. Système selon la revendication 5, dans lequel la seconde unité de fixation (4) présente en outre un second trou traversant (42) configuré pour que le système soit fixé à un tissu.

8. Système selon la revendication 6, dans lequel une direction étendue de la seconde fente est parallèle à une direction longitudinale de la barre de reconstruction de thorax.

9. Système selon la revendication 1, dans lequel la barre de reconstruction de thorax présente une structure en forme d'arc.

10. Système selon la revendication 5, dans lequel la seconde unité de fixation présente une structure en forme d'arc et la seconde unité de fixation est hermétique pour empêcher le tissu humain de migrer dans la seconde unité de fixation.

11. Système selon la revendication 1, dans lequel la première fente est allongée dans le sens de la longueur de la barre de reconstruction de thorax.

12. Système selon la revendication 11, dans lequel la première unité de fixation s'étend dans le sens de la longueur.

13. Système selon la revendication 6, dans lequel la seconde fente est allongée dans le sens de la longueur de la barre de reconstruction de thorax.

14. Système selon la revendication 13, dans lequel la seconde unité de fixation s'étend dans le sens de la longueur.
